# FASCICULE DE BREVET EUROPEEN

(11) **EP 4 039 168 B1**
(45) Date de publication et mention de la délivrance du brevet: **20.12.2023**
(21) Numéro de dépôt: 22154868.8
(22) Date de dépôt: 02.02.2022
(51) Int. Cl.: A61B 1/06, A61B 1/00, A61B 1/31, A61B 1/317, A61B 1/267

(54) **SOURCE DE LUMIERE POUR ENDOSCOPE**
LICHTQUELLE FÜR ENDOSKOP
LIGHT SOURCE FOR ENDOSCOPE

(30) Priorité: 03.02.2021 FR 2101021
(43) Date de publication de la demande: 10.08.2022
(73) Titulaire: Delmont Imaging, 13600 La Ciotat (FR)
(72) Inventeur: BARAN, Stéphane, 83330 Le Beausset (FR); LESPINASSE, Cédric, 13600 La Ciotat (FR); VOLPATTO, Laurent, 13011 Marseille (FR); GREFFEUILLE, Robin, 13600 La Ciotat (FR)
(74) Mandataire: Oudin, Stéphane

(56) Documents cités:
- EP-A1- 2 912 993
- JP-A- H03 224 534
- US-A- 4 579 419
- US-A1- 2011 018 988
- US-B1- 6 257 741

## Description

### Domaine technique de l'invention

La présente invention appartient au domaine du matériel médical. Plus particulièrement, la présente invention concerne une source de lumière pour endoscope.

### Etat de la technique

Dans le domaine médical, il est connu d'utiliser un endoscope qui permet une visualisation directe d'une zone de l'intérieur d'un patient en vue d'un examen visuel tel que, par exemple, une bronchoscopie, une arthroscopie, une célioscopie, une hystéroscopie ou encore une coloscopie, ou d'un acte de chirurgie invasive tel que, par exemple, un acte de chirurgie viscérale et digestive ou encore de chirurgie ligamentaire.

De manière classique, un endoscope comprend au moins :
- un système optique, fabriqué selon la spécialité à partir de fibre optique, de lentille qui sont ensuite interfacé hors du patient avec un dispositif d'acquisition numérique, ou directement d'un capteur d'image dans la partie distale, et
- un dispositif d'éclairage.

En outre, selon son type, l'endoscope peut être muni de plusieurs canaux servant à l'irrigation, l'aspiration, mais également au travail en autorisant le passage de différents instruments.

Ledit dispositif d'éclairage est classiquement fabriqué à partir de fibres optiques, et est connecté à une source de lumière via un câble de conduction de lumière, ce dernier comportant, d'une part, une interface permettant une connexion à ladite source de lumière et, d'autre part, un raccord permettant une connexion à l'endoscope ou à une caméra. Un dispositif d'éclairage de ce type est notamment divulgué dans la demande de brevet américain US 6 257 741.

L'un des inconvénients majeurs de cette configuration d'endoscopes réside dans le fait qu'il existe plusieurs fabricants de câbles de conduction de lumière et que chacun de ces câbles comporte à l'une de ses extrémités une interface spécifique permettant une connexion à une source de lumière du même fabricant, ce qui a pour conséquence pour un établissement de soin de devoir posséder uniquement les câbles de conduction de lumière compatibles avec leurs sources de lumière correspondantes, et donc de devoir changer l'intégralité des câbles de conduction de lumière en cas de changement de fabriquant de source de lumière. Par ailleurs, si un câble de conduction de lumière ou source de lumière d'un fabricant est défectueux, il ne sera pas possible de le remplacer par un câble de conduction de lumière ou source de lumière d'un autre fabricant, car il n'y a pas de compatibilité mécanique entre les produits des différents fabricants.

Pour pallier cet inconvénient, certains fabricants ont mis au point des connecteurs multiples aptes à être connectés en saillie sur leur source de lumière et à recevoir tous les types d'interfaces de câbles de conduction de lumière fabriqués par les 3 principaux fabricants.

Si ces connecteurs multiples permettent certes d'assurer la compatibilité mécanique dans la majorité des cas, ils présentent toutefois les inconvénients suivants :
- une ergonomie médiocre avec ces connecteurs multiples en saillie sur la source de lumière pouvant représenter un risque d'erreur et de détérioration du matériel, et
- une perte importante de puissance lumineuse due au mauvais positionnement de l'extrémité distale de l'interface du câble de conduction de lumière reçu sur lesdits connecteurs multiples en saillie par rapport à la source lumineuse disposée à l'intérieur de la source de lumière.

### Résumé de l'invention

Le but de la présente invention est donc de fournir une source de lumière compatible mécaniquement avec les câbles de conduction de lumière proposés par les principaux fabricants, présentant une bonne ergonomie, c'est-à-dire sans élément en saillie, et garantissant un niveau de puissance lumineuse optimal.

Conformément à l'invention, il est donc proposé une source de lumière agencée pour recevoir un câble de conduction de lumière muni d'une des première, deuxième et troisième interfaces pour acheminer de la lumière destinée à un endoscope, ladite première interface comportant au moins une gorge circulaire au voisinage de son extrémité proximale et une portion cylindrique disposée au voisinage de son milieu, ladite deuxième interface comportant au moins une bague déformable élastiquement disposée en surépaisseur au voisinage du milieu de ladite deuxième interface et une portion cylindrique au voisinage de son extrémité distale, ladite troisième interface comportant au moins une gorge circulaire au voisinage de son extrémité distale et une portion cylindrique disposée au voisinage de son extrémité proximale, ladite source de lumière comportant au moins un carter contentant au moins un organe de focalisation du faisceau lumineux émis par des moyens lumineux et étant remarquable en ce que le carter contient au moins un dispositif de connexion comprenant au moins de l'extérieur vers l'intérieur du carter :
- un orifice aménagé sur la façade avant dudit carter,
- un premier support disposé du côté de la face arrière de ladite façade avant,
- un coulisseau apte à coulisser selon une direction parallèle à la façade avant dudit carter, et
- un deuxième support recevant les moyens lumineux et l'organe de focalisation et en ce que ledit dispositif de connexion est configuré pour permettre, d'une part, de bloquer mécaniquement l'interface du câble de conduction de lumière par un des premier support, coulisseau et deuxième support et, d'autre part, de maintenir ladite interface par au moins un appui obtenu par un autre des premier support, coulisseau et deuxième support, de manière à ce que l'extrémité distale de ladite interface soit toujours parfaitement positionnée et maintenue par rapport audit organe de focalisation.

De manière avantageuse, le premier support comporte un orifice le traversant de part en part et aménagé de sorte à être dans le prolongement de l'orifice de la façade avant, un lamage aménagé sur sa face avant coaxialement à son orifice et au moins une butée entièrement rétractable dans un logement radial aménagé dans la paroi interne dudit lamage et associée à un ressort de poussée tendant à pousser ladite butée vers l'intérieur dudit lamage, ladite butée étant agencée pour coopérer avec la gorge de la première interface pour assurer le blocage mécanique de cette dernière, et ledit orifice étant agencé pour recevoir de façon ajustée la portion cylindrique de la troisième interface afin de former au moins un appui de cette dernière.

Le premier support reçoit de préférence un détecteur de proximité permettant de détecter la présence d'une des première, deuxième et troisième interfaces dans son orifice et d'autoriser la mise en marche des moyens lumineux.

De manière avantageuse, le coulisseau comporte un premier orifice, un deuxième orifice et un troisième orifice le traversant de part en part et disposés de sorte que leurs axes longitudinaux respectifs soient parallèles entre eux et appartiennent à un même plan contenant les axes longitudinaux des orifices respectifs de la façade avant et du premier support, ledit premier orifice étant agencé pour coincer la bague déformable de la deuxième interface pour assurer le blocage mécanique de cette dernière, ledit deuxième orifice étant agencé pour recevoir de façon ajustée la portion cylindrique de la première interface afin de former au moins un appui de cette dernière.

Chacun des premier, deuxième et troisième orifices du coulisseau comporte de préférence un chanfrein de guidage permettant de faciliter une insertion dans l'orifice associé.

Selon un mode de réalisation avantageux, le coulisseau comporte un taraudage le traversant de part en part et dont l'axe longitudinal s'étendant perpendiculairement et en dessous des axes longitudinaux des premier, deuxième et troisième orifices, ledit taraudage coopérant avec une vis sans fin mise en mouvement de rotation par un moteur électrique pour faire translater ledit coulisseau.

De manière avantageuse, le deuxième support comporte un orifice le traversant de part en part et aménagé de sorte à être dans le prolongement de l'orifice de la façade avant dudit carter, et au moins une butée entièrement rétractable dans un logement radial aménagé dans la paroi interne de l'orifice et associée à un ressort de poussée tendant à pousser ladite butée vers l'intérieur dudit orifice, ladite butée étant agencée pour, d'une part, coopérer avec la gorge de la troisième interface pour assurer le blocage mécanique de cette dernière et, d'autre part, coopérer avec la portion cylindrique de la deuxième interface afin de former au moins un appui de cette dernière.

Le deuxième support comporte de préférence un taraudage aménagé sur sa face arrière coaxialement à son orifice et permettant la fixation de l'organe de focalisation.

Ledit deuxième support comporte avantageusement une bague d'arrêt disposée au fond de son orifice et contre laquelle l'extrémité distale de chacune des deuxième et troisième interfaces vient en appui lors de la connexion de cette dernière dans le dispositif de connexion de la source de lumière.

La source de lumière selon l'invention possède avantageusement une fonction traitement des images captées par l'endoscope associé et qu'elle comporte un organe de raccordement agencé pour permettre le raccordement du câble vidéo dudit endoscope et étant disposé au moins en partie sur la façade avant dudit carter.

### Brève description des figures

D'autres avantages et caractéristiques ressortiront mieux de la description qui va suivre d'un mode d'exécution de l'invention, en référence aux figures annexées sur lesquelles :
[Fig 1] est une vue en perspective d'une source de lumière selon l'invention avec les trois types d'interfaces proposées par les principaux fabricants de câbles de conduction de lumière,
[Fig 2] est une vue de face du dispositif de source de lumière de la figure 1,
[Fig. 3] est une vue de coupe verticale partielle selon l'axe A - A' de la source de lumière de la figure 2 recevant un premier type d'interface,
[Fig. 4] est une vue de coupe verticale partielle selon l'axe A - A' de la source de lumière de la figure 2 recevant un deuxième type d'interface,
[Fig 5] est une vue de coupe verticale partielle selon l'axe A - A' de la source de lumière de la figure 2 recevant un troisième type d'interface,
[Fig 6] est une vue de dessus partiellement coupée de la source de lumière de la figure 1,
[Fig. 7] est une vue en perspective d'un coulisseau de la source de lumière de la figure 6,
[Fig. 8] est une vue de face du coulisseau de la figure 7,
[Fig. 9] est une vue arrière du coulisseau de la figure 7.

### Description des modes de réalisation

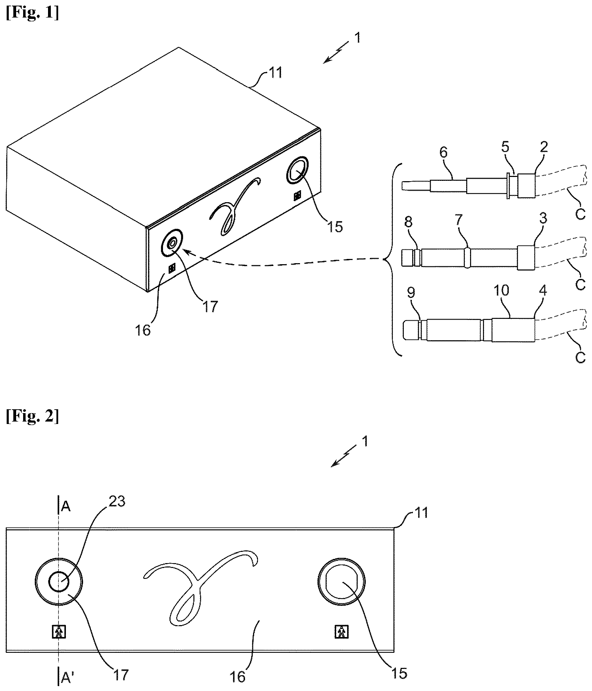

Sur les figures 1 à 6, on a représenté une source de lumière 1 selon l'invention agencé pour recevoir un câble C de conduction de lumière pouvant être de différents diamètre et/ou longueur et étant muni d'une des interfaces 2,3,4 pour acheminer de la lumière destinée à un endoscope à vocation médicale ou à une caméra associée.

Ces interfaces 2,3,4, qui correspondent chacun à l'un des types d'interfaces proposés par les trois principaux fabricants de câbles de conduction de lumière (c'est-à-dire ceux qui représentent à ce jour plus 85% du marché), sont de forme proche : longueur sensiblement égale et une forme globalement tubulaire, mais ils comportent toutefois un certain nombre de différences.

Ainsi, l'interface 2 du premier type, nommée ci-après première interface 2, comporte au moins une gorge circulaire 5 au voisinage de son extrémité proximale et une portion cylindrique 6 disposée au voisinage du milieu de la première interface 2.

On désigne ici par "voisinage du milieu ou d'une extrémité d'une interface" tout élément ou partie d'un élément de l'interface situé pas uniquement strictement au milieu ou à une extrémité de cette dernière, mais également tout élément ou partie d'un élément de l'interface situé à une distance dudit milieu ou de ladite extrémité correspondant au plus à 30% de la longueur totale de ladite interface.

Par ailleurs, l'interface 3 du deuxième type, nommée ci-après deuxième interface 3, comporte au moins une bague déformable 7 élastiquement disposée en surépaisseur au voisinage du milieu de ladite deuxième interface 3 et une portion cylindrique 8 au voisinage de son extrémité distale.

Enfin, l'interface 4 du troisième type, nommée ci-après troisième interface 4, comporte au moins une gorge circulaire 9 au voisinage de son extrémité distale et une portion cylindrique 10 disposée au voisinage de son extrémité proximale.

Selon le mode de réalisation représenté partiellement, la source de lumière 1 comporte un carter 11 contentant au moins :
- des moyens lumineux 12 du type à diodes électroluminescentes,
- un organe de focalisation 13 apte à concentrer le faisceau lumineux émis par lesdits moyens lumineux 12,
- une carte électronique permettant, d'une part, en association, le cas échéant, avec des organes de commande, de gérer l'alimentation et de régler la puissance desdits moyens lumineux 12 et, d'autre part, d'acquérir et de traiter les images captées par un objectif associé à un capteur d'image, avantageusement du type CMOS, et logé dans l'embout distal d'un endoscope associé ou dans une caméra associée à un endoscope et transmises par un câble vidéo,
- une source d'alimentation en énergie électrique ou des moyens de raccordement à un réseau électrique, et
- un dispositif de connexion 14 apte à recevoir une des première, deuxième et troisième interfaces 2,3,4 pour transmettre la lumière sortant de l'organe de focalisation 13 audit endoscope ou à ladite caméra par l'intermédiaire du câble C de conduction de lumière associé, et un organe de raccordement 15 agencé pour permettre le raccordement du câble vidéo dudit endoscope ou de ladite caméra, les dispositif de connexion 14 et organe de raccordement 15 étant disposés au moins en partie sur la façade avant 16 dudit carter 11.

On désigne ici par "avant" tout élément ou partie d'élément de la source de lumière 1 selon l'invention disposé du côté d'un opérateur lors d'une utilisation normale de cette dernière et par "arrière" tout élément ou partie d'élément de ladite source de lumière 1 disposé du côté opposé.

Les moyens lumineux 12 pourront être d'un tout autre type tel que, par exemple, lampe halogène ou encore lampe au xénon, sans sortir du cadre de la présente invention.

Par ailleurs, on comprend bien que, selon un mode de réalisation simplifié non représenté, la source de lumière 1 pourra ne pas posséder de fonction acquisition et traitement des images captées par l'endoscope associé ou la caméra associée. Dans cette hypothèse, la source de lumière 1 comprendra seulement le dispositif de connexion 14 et une carte électronique, dans une version moins complexe, permettant de ne gérer que l'alimentation et de régler la puissance de ladite source lumineuse.

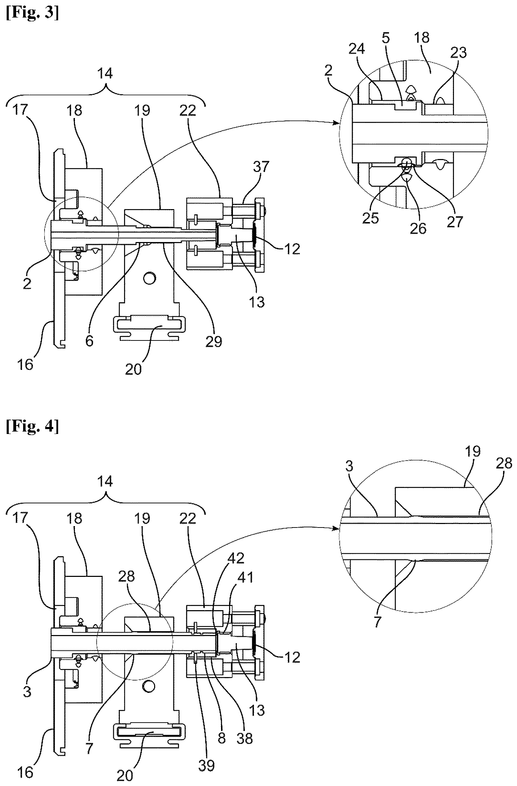

En référence aux figures 3 à 5, pour être en mesure de recevoir n'importe lequel des première, deuxième et troisième interfaces 2,3,4, le dispositif de connexion 14 de la source de lumière 1 comprend au moins de l'extérieur vers l'intérieur du carter 11 de ladite source de lumière 1 :
- un orifice 17 aménagé sur la façade avant 16 dudit carter 11 perpendiculairement à cette dernière, et permettant l'insertion d'une des première, deuxième et troisième interfaces 2,3,4 dans le carter 11,
- un premier support 18 avantageusement disposé du côté de la face arrière de ladite façade avant 16 et étant solidaire ou non de cette dernière,
- un coulisseau 19 apte à coulisser à l'intérieur du carter 11 sur une glissière 20 solidaire du fond 21 du carter 11 selon une direction parallèle à la façade avant 16 dudit carter 11, et
- un deuxième support 22 avantageusement solidaire du fond 21 du carter 11 et recevant les moyens lumineux 12 et l'organe de focalisation 13.

En référence à la figure 3, ledit premier support 18 du dispositif de connexion 14 de la source de lumière 1 comporte un orifice 23 le traversant de part en part et aménagé de sorte à être dans le prolongement de l'orifice 17 de la façade avant 16 dudit carter 11, les orifices 17,23 respectifs de la façade avant 16 et du premier support 18 étant alors coaxiaux. En outre, selon un mode de réalisation avantageux, le premier support 18 comporte un lamage 24 aménagé sur sa face avant coaxialement à son orifice 23.

Ledit premier support 18 comprend en outre au moins une butée 25 entièrement rétractable dans un logement radial 26 aménagé dans la paroi interne du lamage 24 dudit premier support 18, et associée à un ressort de poussée 27 tendant à pousser ladite butée 25 vers l'intérieur dudit lamage 24.

Le premier support 18 du dispositif de connexion 14 peut en outre recevoir un détecteur de proximité, non représenté, par exemple du type à rayonnement infrarouge permettant de détecter la présence d'une des première, deuxième et troisième interfaces 2,3,4 dans son orifice 23 et d'autoriser la mise en marche des moyens lumineux 12.

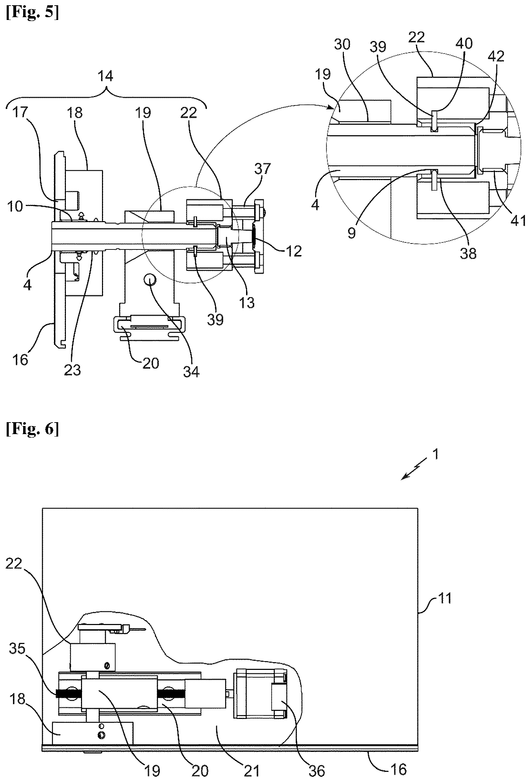

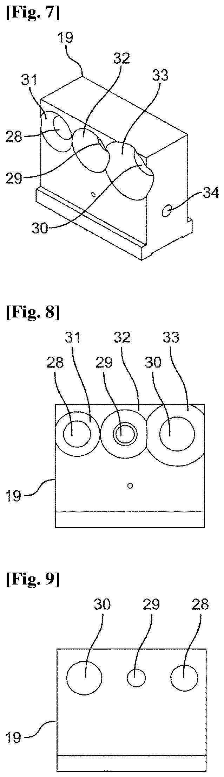

En référence aux figures 6 à 9, le coulisseau 19 a une forme globalement parallélépipédique et comporte un premier orifice 28, un deuxième orifice 29 et un troisième orifice 30 le traversant de part en part et disposés de sorte que leurs axes longitudinaux respectifs soient parallèles entre eux, appartiennent à un même plan contenant les axes longitudinaux des orifices 17,23 respectifs de la façade avant 16 et du premier support 18.

En outre, chacun des premier, deuxième et troisième orifices 28,29,30 du coulisseau 19 comporte un chanfrein 31,32,33 de guidage permettant de faciliter une insertion dans l'orifice associé.

Par ailleurs, le coulisseau 19 comporte un taraudage 34 le traversant de part en part et dont l'axe longitudinal s'étendant perpendiculairement et en dessous des axes longitudinaux des premier, deuxième et troisième orifices 28,29,30. Ledit taraudage 34 coopère avec une vis sans fin 35 mise en mouvement de rotation par un moteur électrique 36 pour faire translater ledit coulisseau 19 le long de la glissière 20 (Cf. figures 5 et 6).

Toutefois, selon un mode de réalisation plus sommaire non représenté, la translation du coulisseau 19 pourra être obtenue de façon manuelle à l'aide, par exemple, d'une languette solidaire dudit coulisseau 19 et traversant la façade avant 16 dudit carter 11.

En référence à la figure 5, comme précédemment décrit, le deuxième support 22 du dispositif de connexion 14 de la source de lumière 1 reçoit les moyens lumineux 12 et l'organe de focalisation 13, lesdits moyens lumineux 12 étant isolés électriquement du reste du deuxième support 22 par des plots 37 en matière isolante telle que, par exemple, du polyétheréthercétone, classiquement désigné par le sigle PEEK (Cf. figure 3).

Ledit deuxième support 22 comporte un orifice 38 le traversant de part en part et aménagé de sorte à être dans le prolongement de l'orifice 17 de la façade avant 16 dudit carter 11, les orifices 17,23,38 respectifs de la façade avant 16, du premier support 18 et du deuxième support 22 étant alors coaxiaux.

A l'instar du premier support 18, ledit deuxième support 22 comprend également au moins une butée 39 entièrement rétractable dans un logement radial 40 aménagé dans la paroi interne de l'orifice 38 dudit deuxième support 22, et associée à un ressort de poussée, non représenté, tendant à pousser ladite butée 39 vers l'intérieur dudit orifice 38.

De plus, le deuxième support 22 comporte également un taraudage 41 aménagé sur sa face arrière coaxialement à son orifice 38 et permettant la fixation de l'organe de focalisation 13.

Enfin, de manière avantageuse, le deuxième support 22 comporte une bague d'arrêt 42 disposée au fond de son orifice 38 et contre laquelle l'extrémité distale de chacune des deuxième et troisième interfaces 3,4 vient en appui lors de la connexion de cette dernière dans le dispositif de connexion 14 de la source de lumière 1, afin de garantir le positionnement de ladite extrémité distale.

Avec la configuration de la source de lumière 1 précédemment décrite, pour connecter une des première, deuxième et troisième interfaces 2,3,4 à cette dernière, on procède de la manière décrite ci-après.

Ainsi, en référence à la figure 3, pour connecter la première interface 2, on translate préalablement le coulisseau 19 pour que son deuxième orifice 29 soit coaxial avec les orifices 23,38 respectifs du premier support 18 et du deuxième support 22. Ensuite, on insère ladite première interface 2 successivement dans l'orifice 17 de la façade avant 16, l'orifice 23 du premier support 18, le deuxième orifice 29 du coulisseau 19 et l'orifice 38 du deuxième support 22, jusqu'à ce que la butée 25 du premier support 18 coopère avec la gorge 5 de la première interface 2 pour assurer le blocage mécanique de ce dernier. Dans cette position, la portion cylindrique 6 de la première interface 2 est insérée de façon ajustée dans le deuxième orifice 29 du coulisseau 19 afin de former un appui pour éviter tout déplacement intempestif de ladite première interface 2 dû au porte-à-faux. Enfin, dans cette position, l'extrémité distale de la première interface 2 est parfaitement positionnée et maintenue par rapport à l'organe de focalisation 13 solidaire du deuxième support 22.

On entend ici par "déplacement intempestif" un déplacement qui tendrait à faire en sorte que l'axe longitudinal d'une des première, deuxième et troisième interfaces 2,3,4 ne soit plus confondu avec les axes longitudinaux de l'orifice 17 de la façade avant 16 et des orifices 23,38 respectifs du premier support 18 et du deuxième support 22.

De même, on désigne ici par "blocage mécanique" le fait qu'une interface soit bloquée dans le dispositif de connexion 14 et ne puisse s'en dégager sans l'intervention d'un opérateur.

Enfin, l'extrémité distale d'une interface est parfaitement positionnée lorsque sa face transversale se trouve à proximité de la face avant de l'organe de focalisation 13, c'est-à-dire à une distance inférieure ou égale à 1 millimètre, et que l'axe longitudinal de l'interface soit confondu avec les axes longitudinaux des orifices 17, 23,38 respectifs de la façade avant 16, du premier support 18 et du deuxième support 22. Cette position de l'extrémité distale de chacune des première, deuxième et troisième interfaces 2,3,4 garantit un niveau optimal de puissance lumineuse transmise à l'endoscope associé. Par ailleurs, dans l'hypothèse où le deuxième support 22 comporte une bague d'arrêt 42, la position de distale de chacune des deuxième et troisième interfaces 3,4 est assurée par le fait que ladite extrémité distale vient en appui contre ladite bague d'arrêt 42 lors de la connexion de l'une des première, deuxième et troisième interfaces 2,3,4 dans le dispositif de connexion 14 de la source de lumière 1.

De même, en référence à la figure 4, pour connecter la deuxième interface 3, on translate préalablement le coulisseau 19 pour que son premier orifice 28 soit coaxial avec les orifices 23,38 respectifs du premier support 18 et du deuxième support 22. Ensuite, on insère ladite deuxième interface 3 successivement dans l'orifice 17 de la façade avant 16, l'orifice 23 du premier support 18, le premier orifice 28 du coulisseau 19 et l'orifice 38 du deuxième support 22, jusqu'à ce que la bague déformable 7 de la deuxième interface 3 vienne se coincer dans le premier orifice 28 du coulisseau 19 pour assurer le blocage mécanique de ladite deuxième interface 3. Dans cette position, la portion cylindrique 8 de la deuxième interface 3 est maintenu en position par la butée 39 du deuxième support 22 formant un appui pour éviter tout déplacement intempestif de ladite deuxième interface 3 dû au porte-à-faux. Enfin, dans cette position, l'extrémité distale de la deuxième interface 3 est parfaitement positionnée et maintenue par rapport à l'organe de focalisation 13 solidaire du deuxième support 22.

On comprend bien que le fait d'avoir un deuxième support 22 avec plusieurs butées décalées angulairement entre elles garantit un meilleur appui et donc un meilleur maintien en position de la deuxième interface 3.

Enfin, en référence à la figure 5, pour connecter la troisième interface 4, on translate préalablement le coulisseau 19 pour que son troisième orifice 30 soit coaxial avec les orifices 23,38 respectifs du premier support 18 et du deuxième support 22. Ensuite, on insère ladite troisième interface 4 successivement dans l'orifice 17 de la façade avant 16, l'orifice 23 du premier support 18, le troisième orifice 30 du coulisseau 19 et l'orifice 38 du deuxième support 22, jusqu'à ce que la butée 39 du deuxième support 22 coopère avec la gorge 9 de la troisième interface 4 pour assurer le blocage mécanique de ce dernier. Dans cette position, la portion cylindrique 10 de la troisième interface 4 est insérée de façon ajustée dans l'orifice 23 du premier support 18 afin de former un appui pour éviter tout déplacement intempestif de ladite troisième interface 4 dû au porte-à-faux. Enfin, dans cette position, l'extrémité distale de la troisième interface 4 est parfaitement positionnée et maintenue par rapport à l'organe de focalisation 13 solidaire du deuxième support 22.

L'homme du métier n'aura aucune difficulté à déterminer les positions et dimensions des différents éléments constitutifs du dispositif de connexion 14 de la source de lumière 1 en fonction des formes et dimensions de chacune des première, deuxième et troisième interfaces 2,3,4.

Enfin, on comprend bien qu'avec cette configuration, quel que soit le type d'interface du câble C de conduction de lumière utilisé, à savoir une des première, deuxième et troisième interfaces 2,3,4, le dispositif de connexion 14 permet, d'une part, de bloquer mécaniquement ce dernier par un des premier support 18, coulisseau 19 et deuxième support 22 et, d'autre part, de le maintenir par au moins un appui obtenu un autre des premier support 18, coulisseau 19 et deuxième support 22, de manière à ce que l'extrémité distale de l'interface utilisé soit toujours parfaitement positionnée et maintenue par rapport à l'organe de focalisation 13 solidaire du deuxième support 22.

De plus, on comprend bien qu'avec cette configuration, quel que soit le type d'interface du câble C de conduction de lumière utilisé, à savoir une des première, deuxième et troisième interfaces 2,3,4, le dispositif de connexion 14 possède une très bonne ergonomie, car il n'a besoin que d'un seul orifice 17 aménagé sur la façade avant 16 dudit carter 11 et ne possède aucun élément en saillie sur ladite façade avant 16.

La source de lumière 1 conforme à l'invention trouve une application particulière pour l'endoscopie médicale et chirurgicale. Toutefois, il est évident que la source de lumière 1 peut également être utilisé dans le domaine de l'endoscopie à vocation industrielle.

Enfin, il va de soi que les exemples de source de lumière 1 conformes à l'invention qui viennent d'être décrits ne sont que des illustrations particulières, en aucun cas limitatives de l'invention.

## Revendications

1. Source de lumière (1) agencée pour recevoir un câble (C) de conduction de lumière muni d'une des première, deuxième et troisième interfaces (2,3,4) pour acheminer de la lumière destinée à un endoscope, ladite première interface (2) comportant au moins une gorge circulaire (5) au voisinage de son extrémité proximale et une portion cylindrique (6) disposée au voisinage de son milieu, ladite deuxième interface (3) comportant au moins une bague déformable (7) élastiquement disposée en surépaisseur au voisinage du milieu de ladite deuxième interface (3) et une portion cylindrique (8) au voisinage de son extrémité distale, ladite troisième interface (4) comportant au moins une gorge circulaire (9) au voisinage de son extrémité distale et une portion cylindrique (10) disposée au voisinage de son extrémité proximale, ladite source de lumière (1) comportant au moins un carter (11) contentant au moins un organe de focalisation (13) du faisceau lumineux émis par des moyens lumineux (12) et étant **caractérisée en ce que** le carter (11) contient au moins un dispositif de connexion (14) comprenant au moins de l'extérieur vers l'intérieur du carter (11) :
- un orifice (17) aménagé sur la façade avant (16) dudit carter (11),
- un premier support (18) disposé du côté de la face arrière de ladite façade avant (16),
- un coulisseau (19) apte à coulisser selon une direction parallèle à la façade avant (16) dudit carter (11), et
- un deuxième support (22) recevant les moyens lumineux (12) et l'organe de focalisation (13)
et **en ce que** ledit dispositif de connexion (14) est configuré pour permettre, d'une part, de bloquer mécaniquement l'interface du câble (C) de conduction de lumière par un des premier support (18), coulisseau (19) et deuxième support (22) et, d'autre part, de maintenir ladite interface par au moins un appui obtenu par un autre des premier support (18), coulisseau (19) et deuxième support (22), de manière à ce que l'extrémité distale de ladite interface soit toujours parfaitement positionnée et maintenue par rapport audit organe de focalisation (13).

2. Source de lumière (1) selon la revendication 1 **caractérisée en ce que** le premier support (18) comporte un orifice (23) le traversant de part en part et aménagé de sorte à être dans le prolongement de l'orifice (17) de la façade avant (16), un lamage (24) aménagé sur sa face avant coaxialement à son orifice (23) et au moins une butée (25) entièrement rétractable dans un logement radial (26) aménagé dans la paroi interne dudit lamage (24) et associée à un ressort de poussée (27) tendant à pousser ladite butée (25) vers l'intérieur dudit lamage (24), ladite butée (25) étant agencée pour coopérer avec la gorge (5) de la première interface (2) pour assurer le blocage mécanique de cette dernière, et ledit orifice (23) étant agencé pour recevoir de façon ajustée la portion cylindrique (10) de la troisième interface (4) afin de former au moins un appui de cette dernière.

3. Source de lumière (1) selon la revendication 2 **caractérisée en ce que** le premier support (18) reçoit un détecteur de proximité permettant de détecter la présence d'une des première, deuxième et troisième interfaces (2,3,4) dans son orifice (23) et d'autoriser la mise en marche des moyens lumineux (12).

4. Source de lumière (1) selon l'une quelconque des revendications 1 à 3 **caractérisée en ce que** le coulisseau (19) comporte un premier orifice (28), un deuxième orifice (29) et un troisième orifice (30) le traversant de part en part et disposés de sorte que leurs axes longitudinaux respectifs soient parallèles entre eux et appartiennent à un même plan contenant les axes longitudinaux des orifices (17,23) respectifs de la façade avant (16) et du premier support (18), ledit premier orifice (28) étant agencé pour coincer la bague déformable (7) de la deuxième interface (3) pour assurer le blocage mécanique de cette dernière, et ledit deuxième orifice (29) étant agencé pour recevoir de façon ajustée la portion cylindrique (6) de la première interface (2) afin de former au moins un appui de cette dernière.

5. Source de lumière (1) selon la revendication 4 **caractérisée en ce que** chacun des premier, deuxième et troisième orifices (28,29,30) du coulisseau (19) comporte un chanfrein (31,32,33) de guidage permettant de faciliter une insertion dans l'orifice associé.

6. Source de lumière (1) selon l'une quelconque des revendications 4 ou 5 **caractérisée en ce que** le coulisseau (19) comporte un taraudage (34) le traversant de part en part et dont l'axe longitudinal s'étendant perpendiculairement et en dessous des axes longitudinaux des premier, deuxième et troisième orifices (28,29,30), ledit taraudage (34) coopérant avec une vis sans fin (35) mise en mouvement de rotation par un moteur électrique (36) pour faire translater ledit coulisseau (19).

7. Source de lumière (1) selon l'une quelconque des revendications 1 à 6 **caractérisée en ce que** le deuxième support (22) comporte un orifice (38) le traversant de part en part et aménagé de sorte à être dans le prolongement de l'orifice (17) de la façade avant (16) dudit carter (11), et au moins une butée (39) entièrement rétractable dans un logement radial (40) aménagé dans la paroi interne de l'orifice (38), et associée à un ressort de poussée tendant à pousser ladite butée vers l'intérieur dudit orifice (38), ladite butée (39) étant agencée pour, d'une part, coopérer avec la gorge (9) de la troisième interface (4) pour assurer le blocage mécanique de cette dernière et, d'autre part, coopérer avec la portion cylindrique (8) de la deuxième interface (3) afin de former au moins un appui de cette dernière.

8. Source de lumière (1) selon la revendication 7 **caractérisée en ce que** le deuxième support (22) comporte un taraudage (41) aménagé sur sa face arrière coaxialement à son orifice (38) et permettant la fixation de l'organe de focalisation (13).

9. Source de lumière (1) selon l'une quelconque des revendications 7 ou 8 **caractérisée en ce que** le deuxième support (22) comporte une bague d'arrêt (42) disposée au fond de son orifice (38) et contre laquelle l'extrémité distale de chacune des deuxième et troisième interfaces (3,4) vient en appui lors de la connexion de cette dernière dans le dispositif de connexion (14) de la source de lumière (1).

10. Source de lumière (1) selon l'une quelconque des revendications 1 à 9 **caractérisée en ce qu'**elle possède une fonction traitement des images captées par l'endoscope associé et qu'elle comporte un organe de raccordement (15) agencé pour permettre le raccordement du câble vidéo dudit endoscope et étant disposé au moins en partie sur la façade avant (16) dudit carter (11).

## Patentansprüche

1. Lichtquelle (1), die dazu angeordnet ist, ein Lichtleitungskabel (C) aufzunehmen, das mit einer der ersten, einer zweiten und einer dritten Schnittstelle (2, 3, 4) versehen ist, um Licht an ein Endoskop weiterzuleiten, wobei die erste Schnittstelle (2) mindestens eine kreisförmige Nut (5) in der Nähe ihres proximalen Endes und einen zylindrischen Abschnitt (6) umfasst, der in der Nähe ihrer Mitte angeordnet ist, wobei die zweite Schnittstelle (3) mindestens einen verformbaren Ring (7), der elastisch als Wulst in der Nähe der Mitte der zweiten Schnittstelle (3) angeordnet ist, und einen zylindrischen Abschnitt (8) in der Nähe ihres distalen Endes umfasst, wobei die dritte Schnittstelle (4) mindestens eine kreisförmige Nut (9) in der Nähe ihres distalen Endes und einen zylindrischen Abschnitt (10) umfasst, der in der Nähe ihres proximalen Endes angeordnet ist, wobei die Lichtquelle (1) mindestens ein Gehäuse (11) umfasst, das mindestens ein Fokussierungselement (13) des von dem Leuchtmittel (12) emittierten Lichtstrahls enthält und **dadurch gekennzeichnet ist, dass** das Gehäuse (11) mindestens eine Verbindungsvorrichtung (14) enthält, die mindestens von der Außenseite zur Innenseite des Gehäuses (11) Folgendes umfasst:
- eine Öffnung (17), die an der Frontplatte (16) des Gehäuses (11) angebracht ist,
- einen ersten Träger (18), der auf der Rückseite der Frontplatte (16) angeordnet ist,
- ein Gleitstück (19), das dazu ausgelegt ist, parallel zur Frontplatte (16) des Gehäuses (11) zu gleiten, und
- einen zweiten Träger (22), der das Leuchtmittel (12) und das Fokussierungselement (13) aufnimmt,
und dadurch, dass die Verbindungsvorrichtung (14) dazu konfiguriert ist, zu gestatten, dass einerseits die Schnittstelle des Lichtleitungskabels (C) durch einen von dem ersten Träger (18), dem Gleitstück (19) und dem zweiten Träger (22) mechanisch blockiert wird und andererseits die Schnittstelle durch mindestens ein Stützelement gehalten wird, das durch ein anderes von dem ersten Träger (18), dem Gleitstück (19) und dem zweiten Träger (22) erhalten wird, so dass das distale Ende der Schnittstelle immer perfekt positioniert und im Verhältnis zum Fokussierungselement (13) gehalten wird.

2. Lichtquelle (1) nach Anspruch 1, **dadurch gekennzeichnet, dass** der erste Träger (18) eine Öffnung (23), die durch ihn verläuft und derart angebracht ist, dass sie sich in der Verlängerung der Öffnung (17) der Frontplatte (16) befindet, eine Senkung (24), die auf seiner Vorderseite koaxial zu seiner Öffnung (23) angebracht ist, und mindestens einen Anschlag (25) umfasst, der vollständig in eine radiale Aufnahme (26) zurückziehbar ist, die in der Innenwand der Senkung (24) angebracht ist und mit einer Druckfeder (27) in Verbindung steht, die dazu neigt, den Anschlag (25) in das Innere der Senkung (24) zu drücken, wobei der Anschlag (25) dazu angeordnet ist, mit der Nut (5) der ersten Schnittstelle (2) zusammenzuwirken, um die mechanische Blockierung der letzteren sicherzustellen, und wobei die Öffnung (23) dazu angeordnet ist, den zylindrischen Abschnitt (10) der dritten Schnittstelle (4) in angepasster Weise aufzunehmen, um mindestens ein Stützelement für letztere zu bilden.

3. Lichtquelle (1) nach Anspruch 2, **dadurch gekennzeichnet, dass** der erste Träger (18) einen Näherungsdetektor aufnimmt, der es gestattet, das Vorhandensein einer der ersten, der zweiten und der dritten Schnittstelle (2, 3, 4) in seiner Öffnung (23) zu detektieren und das Einschalten des Leuchtmittels (12) zu ermöglichen.

4. Lichtquelle (1) nach einem der Ansprüche 1 bis 3, **dadurch gekennzeichnet, dass** das Gleitstück (19) eine erste Öffnung (28), eine zweite Öffnung (29) und eine dritte Öffnung (30) umfasst, die durchgehend durch es verlaufen und derart angeordnet sind, dass ihre jeweiligen Längsachsen parallel zueinander sind und zur gleichen Ebene gehören, die die Längsachsen der jeweiligen Öffnungen (17, 23) der Frontplatte (16) und des ersten Trägers (18) enthält, wobei die erste Öffnung (28) dazu angeordnet ist, den verformbaren Ring (7) der zweiten Schnittstelle (3) einzuklemmen, um die mechanische Blockierung der letzteren sicherzustellen, und wobei die zweite Öffnung (29) dazu angeordnet ist, den zylindrischen Abschnitt (6) der ersten Schnittstelle (2) in angepasster Weise aufzunehmen, um mindestens ein Stützelement für letztere zu bilden.

5. Lichtquelle (1) nach Anspruch 4, **dadurch gekennzeichnet, dass** jede der ersten, der zweiten und der dritten Öffnung (28, 29, 30) des Gleitstücks (19) eine Führungsfase (31, 32, 33) umfasst, die es gestattet, ein Einführen der zugehörigen Öffnung zu erleichtern.

6. Lichtquelle (1) nach einem der Ansprüche 4 oder 5, **dadurch gekennzeichnet, dass** das Gleitstück (19) ein Gewinde (34) umfasst, das durchgehend durch ihn verläuft und dessen Längsachse sich senkrecht und unterhalb der Längsachsen der ersten, der zweiten und der dritten Öffnung (28, 29, 30) erstreckt, wobei das Gewinde (34) mit einer Endlosschraube (35) zusammenwirkt, die von einem Elektromotor (36) in Drehbewegung versetzt wird, um das Gleitstück (19) zu verschieben.

7. Lichtquelle (1) nach einem der Ansprüche 1 bis 6, **dadurch gekennzeichnet, dass** der zweite Träger (22) eine Öffnung (38), die durchgehend durch ihn verläuft und derart angebracht ist, dass sie in der Verlängerung der Öffnung (17) der Frontplatte (16) des Gehäuses (11) ist, und mindestens einen Anschlag (39) umfasst, der vollständig in eine radiale Aufnahme (40) zurückziehbar ist, die in der Innenwand der Öffnung (38) angebracht ist und mit einer Druckfeder in Verbindung steht, die dazu neigt, den Anschlag in das Innere der Öffnung (38) zu drücken, wobei der Anschlag (39) dazu angeordnet ist, einerseits mit der Nut (9) der dritten Schnittstelle (4) zusammenzuwirken, um die mechanische Blockierung letzterer sicherzustellen, und andererseits mit dem zylindrischen Abschnitt (8) der zweiten Schnittstelle (3) zusammenzuwirken, um mindestens ein Stützelement für letztere zu bilden.

8. Lichtquelle (1) nach Anspruch 7, **dadurch gekennzeichnet, dass** der zweite Träger (22) ein Gewinde (41) umfasst, das an seiner Rückseite koaxial zu seiner Öffnung (38) angebracht ist und das Befestigen des Fokussierelements (13) gestattet.

9. Lichtquelle (1) nach einem der Ansprüche 7 oder 8, **dadurch gekennzeichnet, dass** der zweite Träger (22) einen Sicherungsring (42) umfasst, der am Boden seiner Öffnung (38) angeordnet ist und an dem das distale Ende jeweils der zweiten und der dritten Schnittstelle (3,4) zum Anliegen kommt, wenn letztere mit der Verbindungsvorrichtung (14) der Lichtquelle (1) verbunden werden.

10. Lichtquelle (1) nach einem der Ansprüche 1 bis 9, **dadurch gekennzeichnet, dass** sie eine Verarbeitungsfunktion für Bilder besitzt, die vom zugehörigen Endoskop aufgenommen werden, und dass sie ein Anschlusselement (15) umfasst, das dazu angeordnet ist, den Anschluss des Videokabels des Endoskops zu gestatten, und das mindestens teilweise an der Frontplatte (16) des Gehäuses (11) angeordnet ist.

## Claims

1. A light source (1) arranged to receive a light-conducting cable (C) provided with one of first, second and third interfaces (2, 3, 4) for conveying light intended for an endoscope, said first interface (2) including at least one circular groove (5) in the vicinity of its proximal end and a cylindrical portion (6) disposed in the vicinity of its middle, said second interface (3) including at least one elastically deformable ring (7) thickened in the vicinity of the middle of said second interface (3) and a cylindrical portion (8) in the vicinity of its distal end, said third interface (4) comprising at least one circular groove (9) in the vicinity of its distal end and a cylindrical portion (10) disposed in the vicinity of its proximal end, said light source (1) comprising at least one casing (11) containing at least one member for focusing (13) the light beam emitted by light means (12) and being **characterised in that** the casing (11) contains at least one connection device (14) comprising, at least from the outside towards the inside of the casing (11):
- a port (17) arranged on the front panel (16) of said casing (11),
- a first support (18) disposed on the rear face of said front panel (16),
- a slider (19) able to slide along a direction parallel to the front panel (16) of said casing (11), and
- a second support (22) receiving the light means (12) and the focusing member (13)
and **in that** said connection device (14) is configured so as to make it possible, on the one hand, to mechanically block interface of the light-conducting cable (C) via one of the first support (18), slider (19) and second support (22) and, on the other hand, to hold said interface via at least one fulcrum obtained by another of the first support (18), slider (19) and second support (22), so that the distal end of said interface is always perfectly positioned and held with respect to said focusing member (13).

2. The light source (1) according to claim 1, **characterised in that** the first support (18) includes a port (23) passing therethrough from one side to the other and arranged so as to be as an extension of the port (17) of the front panel (16), a counterbore (24) arranged on its front face coaxially with its port (23) and at least one stop (25) fully retractable into a radial housing (26) arranged in the internal wall of said counterbore (24) and associated with a pushing spring (27) tending to push said stop (25) towards the inside of said counterbore (24), said stop (25) being arranged to cooperate with the groove (5) of the first interface (2) to ensure mechanical blocking thereof, and
said port (23) being arranged to fittingly receive the cylindrical portion (10) of the third interface (4) in order to form at least one fulcrum for the same.

3. The light source (1) according to claim 2, **characterised in that** the first support (18) receives a proximity detector for detecting presence of one of the first, second and third interfaces (2, 3, 4) in its port (23) and for authorising the light means (12)to be switched on.

4. The light source (1) according to any of claims 1 to 3, **characterised in that** the slider (19) includes a first port (28), a second port (29) and a third port (30) passing therethrough from one side to the other and disposed so that their respective longitudinal axes are parallel to each other and belong to a same plane containing the longitudinal axes of the ports (17, 23) of the front panel (16) and of the first support (18), said first port (28) being arranged to wedge the deformable ring (7) of the second interface (3) to ensure mechanical blocking thereof, and said second port (29) being arranged to fittingly receive the cylindrical portion (6) of the first interface (2) in order to form at least one fulcrum for the same.

5. The light source (1) according to claim 4, **characterised in that** each of the first, second and third ports (28, 29, 30) of the slider (19) includes a guide chamfer (31, 32, 33) for facilitating insertion into the associated port.

6. The light source (1) according to any of claims 4 or 5, **characterised in that** the slider (19) includes a thread tap (34) passing therethrough from one side to the other, the longitudinal axis of which extends perpendicularly to and below the longitudinal axes of the first, second and third orifices (28, 29, 30), said thread tap (34) cooperating with an endless screw (35) rotatably moved by an electric motor (36) in order to cause said slider (19) to translate.

7. The light source (1) according to any of claims 1 to 6, **characterised in that** the second support (22) includes a port (38) passing therethrough from one side to the other and arranged so as to be as an extension of the port (17) of the front panel (16) of said casing (11), and at least one stop (39) fully retractable into a radial housing (40) arranged in the internal wall of the port (38), and associated with a pushing spring tending to push said stop towards the inside of said port (38), said stop (39) being arranged so as, on the one hand, to cooperate with the groove (9) of the third interface (4) in order to ensure mechanical blocking thereof and, on the other hand, to cooperate with the cylindrical portion (8) of the second interface (3) in order to form at least one fulcrum for the same.

8. The light source (1) according to claim 7, **characterised in that** the second support (22) includes a thread tap (41) arranged on its rear face coaxially with its port (38) and allowing the focusing member (13) to be attached.

9. The light source (1) according to any of claims 7 or 8, **characterised in that** the second support (22) includes a stop ring (42) disposed at the bottom of its port (38) and against which the distal end of each of the second and third interfaces (3, 4) bears as a fulcrum upon connecting the same in the connection device (14) of the light source (1).

10. The light source (1) according to any of claims 1 to 9, **characterised in that** it has a function for processing images sensed by the associated endoscope and that it includes a connection member (15) arranged to allow connection of the video cable of said endoscope and being at least partly disposed on the front panel (16) of said casing (11).
